(19) Europäisches Patentamt
European Patent Office
Office européen des brevets

(11) Numéro de publication: **0 003 189**
**B1**

(12) # FASCICULE DE BREVET EUROPEEN

(21) Numéro de dépôt: **79400003.4**

(51) Int. Cl.³: **C 07 C 149/24**

(22) Date de dépôt: **04.01.79**

(54) **Tris(amino-5 thia-3 pentyl)amine et son procédé de préparation**

(30) Priorité: **11.01.78 FR 7800624**

(43) Date de publication de la demande:
**25.07.79 Bulletin 79/15**

(45) Mention de la délivrance du brevet:
**23.07.80 Bulletin 80/15**

(84) Etats contractants désignés:
**BE CH DE GB NL**

(56) Documents cités:
**US - A - 2 304 623**

(73) Titulaire: **Rhone-Poulenc Industries**
**22, avenue Montaigne**
**F - 75008 Paris (FR)**

(72) Inventeur: **Soula, Gérard**
**33, rue Nungesser**
**F - 69330 Meyzieu (FR)**

(74) Mandataire: **Fabre, Madeleine-France**
**Rhone-Poulenc Service Brevets Chimie et Polymères B.P. 753**
**F - 75360 Paris Cedex 08 (FR)**

**EP 0 003 189 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Tris(amino-5 thia-3 pentyl)amine et son procédé de préparation

La présente invention a pour objet, à titre de produit industriel nouveau, la tris(amino-5 thia-3 pentyl)amine et un procédé permettant de préparer ledit produit.

La tris(amino-5 thia-3 pentyl)amine faisant l'objet de la présente invention est un produit de formule:

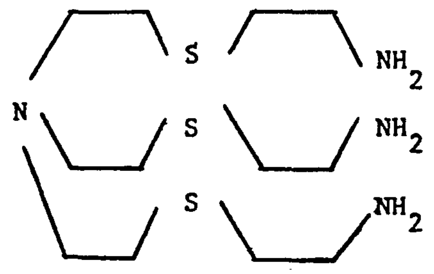

Elle peut être préparée par action de la tris(éthanethiol)amine sur de l'arizidine selon un rapport molaire aziridine/tris(éthanethiol)amine compris entre 3 et 3,5, de préférence entre 3 et 3,2 à une température comprise entre 30 et 80°C, de préférence entre 45 et 80°C, puis séparation de la tris-(amino-5 thia-3 pentyl)amine obtenue.

La réaction se déroule selon le schéma suivant:

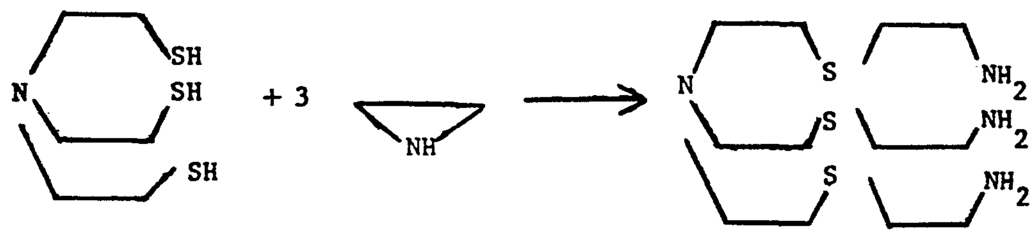


La réaction entre l'aziridine et la tris(éthanethiol)amine est réalisée en présence d'un solvant tel que le méthanol, l'éthanol, le chloroforme, le chlorure de méthylène, le chlorobenzène....cette réaction dure environ de 0,5 à 3 heures.

La tris(éthanethiol)amine à mettre en oeuvre peut être préparée par action du chlorhydrate de trichloréthylamine sur de la thiourée, puis neutralisation du sel formé par une base, selon le mode préparatoire décrit dans J. Chem. Soc — 1947 — pages 320—322 (John Harley-Mason).

La tris(amino-5 thia-3 pentyl)amine faisant l'objet de la présente invention peut être utilisée comme intermédiaire pour la préparation d'additifs pour huiles lubrifiantes.

Les exemples suivants sont donnés à titre indicatif et ne peuvent être considérés comme une limite du domaine et de l'esprit de l'invention.

## EXEMPLE

— *Synthèse de la tris(amino-5 thia-3 pentyl)amine*

Dans un ballon tricol de 500 cm3 équipé d'un agitateur mécanique, d'un thermomètre et d'une ampoule à brome, on introduit 19,7 g, soit 0,1 mole de tris(thioéthanol)amine en solution dans 250 cm3 de méthanol. Le mélange est chauffé à 45°C. On introduit alors 0,3 mole d'aziridine (15,5 ml) diluée dans 20 cm3 de méthanol, en 30 minutes à l'aide de l'ampoule à brome. Le mélange est ensuite porté à la température de reflux du méthanol (64°C) pendant 3 heures et enfin le méthanol est évaporé. On obtient 32 g de produit huileux dont la composition chimique est la suivante:

| | % mesuré | % théorique |
|---|---|---|
| Soufre | 29,5 | 29,4 |
| Azote | 17,1 | 17,2 |
| Carbone | 44,3 | 44,1 |
| Hydrogène | 9,15 | 9,2 |

## Revendications

1. A titre de produit industriel nouveau, la tris(amino-5 thia-3 pentyl)amine.

2. Procédé de préparation de la tris(amino-5 thia-3 pentyl)amine caractérisé en ce que l'on fait réagir de la tris(éthanethiol)amine sur de l'aziridine selon un rapport molaire aziridine/tris(éthanethiol)-amine compris entre 3 et 3,5 à une température comprise entre 30 et 80°C, puis en ce que l'on sépare la tris(amino-5 thia-3 pentyl)amine obtenue.

3. Procédé selon la revendication 2 caractérisé en ce que le rapport molaire aziridine/tris(éthanethiol)amine est compris entre 3 et 3,2.

4. Procédé selon la revendication 2 ou la revendication 3, caractérisé en ce que la réaction entre la tris(éthanethiol)amine et l'aziridine est réalisée à une température comprise entre 45 et 80°C.

5. Procédé selon l'une quelconque des revendications 2 à 4, caractérisé en ce que la réaction entre la tris(éthanethiol)amine et l'aziridine est réalisée en présence d'un solvant choisi parmi le méthanol, l'éthanol, le chloroforme, le chlorure de méthylène, le chlorobenzène.

**Claims**

1. A novel industrial product, tris(5-amino 3-thia pentyl) amine.

2. A process of preparing tris(5-amino 3-thia pentyl)amine characterized in that tris(ethanethiol)-amine is reacted with aziridine in a molar ratio of aziridine to tris(ethane-thiol) amine of between 3 and 3.5 at a temperature of between 30° and 80°C, and in that the tris(5-amino 3-thia pentyl)amine obtained is then separated.

3. A process according to Claim 2, wherein the molar ratio of aziridine to tris(ethanethiol) amine is between 3 and 3.2.

4. A process according to claim 2 or to claim 3, wherein the reaction between the tris(ethanethiol)amine and the aziridine is carried out at a temperature of between 45 and 80°C.

5. A process according to any one of claims 2 to 4 characterized in that the reaction between the tris(ethanethiol)amine and the aziridine is carried out in the presence of a solvent selected from methanol, ethanol, chloroform, methylene chloride, and chlorobenzene.

**Patentansprüche**

1. Tris (5-amino 3-thia pentyl)amin als neues Industrieprodukt.

2. Verfahren zur Herstellung von Tris (5-amino 3-thia pentyl) amin, dadurch gekennzeichnet, dass Tris(äthanthiol)amin mit Aziridin in einem Molverhältnis Aziridin / Tris(äthanthiol)amin von 3 — 3, bei einer Temperatur von 30°—80°C umgesetzt und das erhaltene Tris (5-amino 3-thia pentyl)amin abgetrennt wird.

3. Verfahren nach Anspruch 2, dadurch gekennzeichnet, dass das Molverhältnis Aziridin / Tris-(äthanthiol)amin zwischen 3 und 3,2 liegt.

4. Verfahren nach Anspruch 2 bzw. Anspruch 3, dadurch gekennzeichnet, dass die Reaktion von Tris(äthanthiol)amin mit Aziridin bei einer Temperatur von 45—80°C erfolgt.

5. Verfahren nach einem der Ansprüche 2—4, dadurch gekennzeichnet, dass die Reaktion von Tris(äthanthiol)amin mit Aziridin in Gegenwart eines Lösungsmittels erfolgt, welches unter Methanol, Äthanol, Chloroform, Methylenchlorid,Chlorbenzol gewahlt wird.